## Europäisches Patentamt
## European Patent Office
## Office européen des brevets

(19)

(11) Publication number: **0 208 338**

A2

(12)

# EUROPEAN PATENT APPLICATION

(21) Application number: **86111207.6**

(51) Int.Cl.⁴: **A 61 N 1/40**

(22) Date of filing: **11.01.82**

(30) Priority: **09.01.81 US 223727**

(43) Date of publication of application:
**14.01.87 Bulletin 87/3**

(84) Designated Contracting States:
**AT BE CH DE FR GB IT LI LU NL SE**

⅞¼Publication number of the earlier application
in accordance with Art. 76 EPC: **0 056 697**

(71) Applicant: **Rand, Robert W.**
**521 N. Bristol Street**
**Los Angeles California 90049(US)**

(71) Applicant: **Snow, Harold D.**
**4201 Noble Avenue**
**Sherman Oaks California 91403(US)**

(71) Applicant: **Elliott, David G.**
**737 W. Starlight Heights**
**La Canada California 91011(US)**

(71) Applicant: **Haskins, Glen M.**
**3811 Rio Hondo Avenue**
**Rosemead California 91770(US)**

(72) Inventor: **Rand, Robert W.**
**521 N. Bristol Street**
**Los Angeles California 90049(US)**

(72) Inventor: **Snow, Harold D.**
**4201 Noble Avenue**
**Sherman Oaks California 91403(US)**

(72) Inventor: **Elliott, David G.**
**737 W. Starlight Heights**
**La Canada California 91011(US)**

(72) Inventor: **Haskins, Glen M.**
**3811 Rio Hondo Avenue**
**Rosemead California 91770(US)**

(74) Representative: **Spencer, Graham Easdale**
**A.A. Thornton & CO Northumberland House 303-306,**
**High Holborn**
**London WC1V 7LE(GB)**

(54) Induction heating apparatus.

(57) An induction heating apparatus comprises an induction heating coil (12) and means (30, 32) for passing an alternating current through the coil to cause induction heating therein, characterised in that at least one further coil (12') is provided adjacent and in axial alignment with the heating coil, the or each further coil (12') being electrically isolated from the means (30, 32) for passing an alternating current and being connected to form a resonant circuit (26) arranged to resonate when alternating current passes through the heating coil (12) thereby to provide additional induction heating.

EP 0 208 338 A2

Croydon Printing Company Ltd.

./..

FIG. 1

- 1 -

## Induction heating apparatus

The present invention is concerned with an induction heating apparatus which is particularly suitable for use in the treatment of neoplasms by hyperthermal necrosis.

It is known to treat aberrant cells in animal bodies (neoplasms) by hyperthermia; this causes necrosis of the neoplasms because the latter are more susceptible to heat than normal cells. Various methods of heating have been proposed for such treatment, for example, heating by means of radio-frequency or microwave radiation, and industive heating using an alternating magnetic field.

A radio frequency or microwave field only causes heating at or adjacent to the surface of a body because of the tendency of the body to absorb radio frequency electromagnetic radiation (the depth to which such radiation penetrates the body depends upon the frequency of the radiation). Such body tissue heating as is caused by radio frequency radiation has two principal components: eddy current heating and dielectric heating. Heating of this type can be achieved in the absence of any foreign material in the tissue being heated.

Induction heating of body tissue as a result of the presence of a material having hysteresis characteristics (referred to as hysteresis heating) involves the application of a magnetic field which is generally unattenuated by tissue. Hence, induction heating can reach any desired depth or level beneath the surface of the tissue. In order to achieve this manner of heating in tissue, it is necessary to locate a material exhibiting magnetic hysteresis close to the site where the heat is required. The term "hysteresis composition" is used hereinafter to describe a composition suitable for introducing such a material into the body. A preferred hysteresis composition for use with the apparatus of the present invention is described in European Patent Application No. 82300125.0.

Induction-hysteresis heating is generally accompanied by some eddy current and dielectric heating, but the amount of eddy current and dielectric heating can be minimized by using a comparatively low frequency alternating magnetic field. The amount of such heating varies directly with the square of the frequency of the field, while the amount of hysteresis heating varies directly with the frequency. Thus, in general, the lower the frequency of the field the less undesired eddy current heating relative to the amount of the desired hysteresis heating. (The frequency, of course, should be sufficiently high to ensure the desired degree of hysteresis heating).

The limited amount that a radio frequency field can normally penetrate body tissue is an important factor limiting the use of this type of heating in the treatment of neoplasms since radio frequency heating can not be utilized effectively in causing hyperthermia in aberrant growths which are so far beneath the surface of tissue that they are not reached by the radio frequency field. The use of such a field to cause hyperthermia at or adjacent to the surface of the skin or at or adjacent to the interior of an incision within the body is limited by another important factor - the problem of selectively concentrating the heat so that neoplasm is heated sufficiently to cause necrosis without related or corresponding heating of adjacent normal tissue.

This can be illustrated by referring to specific temperatures which have previously been recognized to be important in causing necrosis by hyperthermia. It is believed that holding the body tissue temperature at $40^{\circ}C$ generally will not cause necrosis, but a temperature of $42^{\circ}C$ will, if the body tissue is maintained at this temperature for long enough, while a temperature of $60^{\circ}C$ or more causes practically instantaneous necrosis. Body tissue should not, of course, be subjected to temperatures near to $100^{\circ}C$ for even a very short time because of the danger of vaporisation of water; in practice, therefore, the tissue is not normally heated to more than $90^{\circ}C$.

The time necessary to cause necrosis at a given temperature is not directly dependent on the temperature in the usual manner of temperature-dependent reactions; in addition, certain parts of the body or neoplasms are more susceptible to necrosis at an elevated temperature than other parts of the body and/or other neoplasms. Because of these factors, it is impossible to give exact relationships between the time and the temperature necessary to accomplish necrosis of neoplasms. Another factor which is important in considering the amount of hyperthermia to cause necrosis in neoplasm is the tendency of the body and/or a particular portion of the body to serve as a heat insulator which tends to concentrate heat developed, for example, at a specific source.

With radio frequency or microwave hyperthermia, the difficulty in confining the field necessary to cause necrosis of a specific area or region will normally cause necrosis of healthy tissue in an adjacent area or region.  While to a degree this can be counteracted by the use of special electrodes, it is impossible to adequately control radio frequency or microwave heating in many applications so as to avoid damage to adjacent normal cells or tissue.  While it may be possible to improve the concentration of the heating effects achieved with radio frequency or microwave heating by the implantation of a metallic conductor or another similar material in a specific area where concentrated heating is desired, the use of a conductor is not always advantageous because it does not improve the depth of penetration of tissue by the radio frequency field.

The use of induction heating in causing necrosis of neoplasm alleviates several problems involved in radio frequency hyperthermia treatment.  When induction heating is used for this purpose, it is necessary to utilize an invasive technique to locate a material having a magnetic permeability greater than unity and capable of exhibiting hysteresis losses, for example, ferromagnetic particles such as a conventional ferrite or conventional iron or steel powders, in or immediately adjacent to the neoplastic growth.  The application of an alternating magnetic field results in hysteresis heating within the magnetic material itself, which avoids the problem of containing or limiting the heated area obtained which arises when using a radio frequency field.  Because of the penetration of low frequency magnetic fields into body tissue, it is possible to use a magnetic material well beneath the surface of the body.

Prior proposals for induction heating to cause necrosis suffer from several disadvantages.  In particular, there is a general lack of induction heating equipment which is specifically adapted for treating a human or animal body;  prior induction heating coils are inefficient for this purpose as they are not specifically designed for providing a very intense AC magnetic field over a comparatively large volume so as to cause heating in a specific, very limited area or region coming within the scope of the magnetic field produced by the coil, in which material capable of hysteresis heating is positioned.

According to the present invention, there is provided an induction heating apparatus which comprises an induction heating coil and means for passing an alternating current through the coil to cause induction heating therein, characterised in that at least one further coil is provided adjacent and in axial alignment with the heating coil, the or each further coil being electrically isolated from the means for passing an alternating current and being connected to form a resonant circuit arranged to resonate when alternating current passes through the heating coil thereby to provide additional induction heating.

The apparatus of the invention can be easily and conveniently constructed at relatively low cost.

In order that the invention may be more fully understood, a preferred embodiment thereof will now be described, by way of example, with reference to the accompanying drawings, in which:

Figure 1 is a diagrammatic view of the essential features of an embodiment of an apparatus in accordance with the present invention; and

Figure 2 is a perspective view showing the configuration of the coils when in use.

As shown in the Figures, apparatus 10 comprises a series of coaxial, identically constructed and dimensioned flat coils 12, 12'. Each of these coils 12, 12' consists of a series of turns (not separately numbered) of an electrically non-conductive tube 14 such as an extruded polyvinyl polymer tube located in two flat, parallel planes and a conductor 16 going through the interior of each tube 14. These conductors 16 are typically multi-stranded wires which are sufficiently small in diameter to permit a cooling liquid such as, for example, a dielectric oil or distilled water to be circulated through the tubes 14 around the conductors 16.

Appropriate conventional fittings 18 are provided at the ends (not separately numbered) of the tubes 14 for the purpose of joining the ends of the tubes 14 to manifolds or distributing pipes 20 used in connection with a coolant storage reservoir 22 and a pump 24. These fittings 18 are formed in a conventional or known manner so that the conductors 16 extend through them so that these conductors 16 may be connected as hereinafter indicated.

In the present invention, each conductor 16 constitutes the electrically "active" part of a coil 12, 12' and is connected into a tank circuit 26 across two separate capacitors 28 of equal capacitance value located in series with one another. All of the coils 12, 12' and the associated capacitors 28 together constitute a tank circuit having a specific resonant frequency.

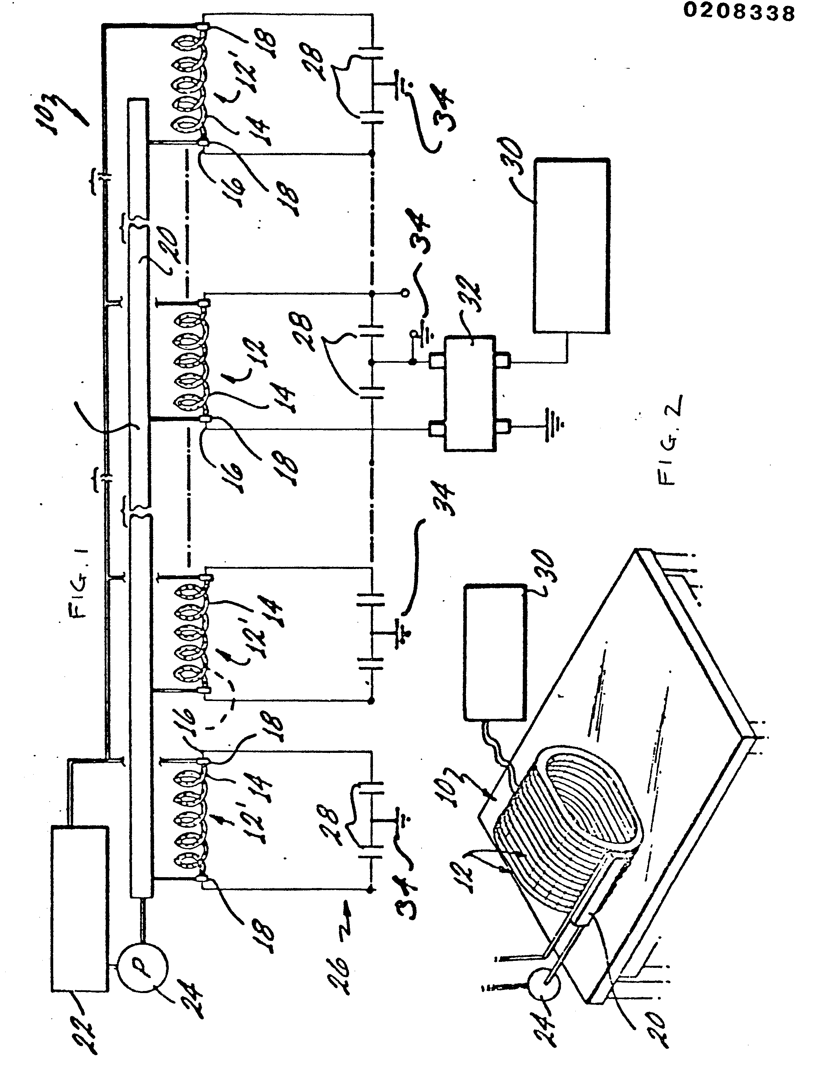

A known adjustable power supply 30 capable of being adjusted so as to operate at various frequencies is used to supply current at the noted resonant frequency to a transformer 32 which in turn is connected to only one of the tank circuits 26. In the particular embodiment illustrated in Figure 1, the transformer 32 is connected across only one of the two capacitors 28 in the tank circuit 26. However, if desired, this transformer 32 can be connected across both of the capacitors 28 in this particular circuit 26.

All of the circuits 26 are grounded by conventional grounds 34. In the interests of safety it is preferred to ground each circuit 26 between the capacitors 28 in each circuit 26 as shown. With the present invention the particular coil 12 which is driven by the power supply 30 and the transformer 32 may be any one of the individual coils used in the complete apparatus 10.

As shown in Figure 2, the coils 12, 12' are assembled closely together in a stack of aligned, identical pancake-type coils and are preferably secured together as a unit by the use of a conventional, appropriate adhesive (not shown). Because they are used in this manner, in effect, the coils 12 constitute a series of closely coupled transformer coils. As a consequence of this, the use of the power supply 30 with a single coil 12 has the effect of driving the remainder of the coils 12' and, of course, the associated tank circuits 26.

In order to achieve the degree of hysteresis required to heat the comparatively small area or region of the body where the hysteresis composition is located, it is necessary to drive the particular circuit 26 connected to the power supply 30 using a comparatively large amount of power. The exact amount of power required will, of course, depend on many factors. At the present time, it is considered that the exact amount of power required to treat various different reasonably related treatments of neoplastic tissue should be judged on an empirical basis taking into consideration the factors which are discussed below.

The frequency of the AC current supplied to the coil 12 and its associated circuit 26 is normally determined on the basis of the factors indicated in the preceding discussion relative to the background of the invention with a view to minimizing eddy current type heating losses while concurrently maximizing the efficiency of the hysteresis heating obtained. It is considered that a frequency of from 1000 to 5000 Hz, preferably about 2000 Hz, initially represents the most desirable balance between the competitive factors which are involved in achieving the desired hyperthermia using the apparatus of the invention and that the individual tank circuits 26 should be formed to resonate

0208338

at a specific frequency and that the power supply 30 should be operated at this frequency.

As power is supplied to a circuit 26 and its associated coil 12, a cooling liquid is supplied to the individual coils 12 in an effort to prevent any temperature rise which would damage these coils or their manner of operation. However, in spite of this measure, some heating will normally occur in the individual conductors 16 in the various coils 12, 12' and in connection with the various associated capacitors 28. Because of such heating, the individual circuits 26 will change slightly as they are used and this in turn will affect the resonant frequency of the complete apparatus.

Because of this, as the complete apparatus 10 is operated, the frequency of the power supply 30 used to drive directly one circuit 26 and its associated coil 12 and the remainder of the circuits 26 and coils 12' indirectly should be adjusted so as to always match the new resonant frequency of the complete collection of circuits 26. In order to further minimize power losses, it is considered highly preferable to utilize as the conductors 16 either wires composed of a twisted plurality of strands of individual wires or conventional Litz wires.

The utilization of such conductors may also be advantageous for another reason. As indicated, during the use of the coils 12, 12' at high power levels, a cooling fluid must be circulated through the tubes 16, in order to prevent damage to either the tubes 14 or melting of the conductors 16. The surface configuration of either a twisted or a Litz wire conductor has been found to be effective in permitting efficient heat transfer from the conductor to the cooling liquid employed.

When using the apparatus 10, power is supplied from the power supply 30 to a connected tank circuit 26 at a frequency matching the resonant frequency of all of the circuits 26 as a unit. When this specific circuit 26 is driven or powered in this manner, it sets up a flux field which couples with the adjacent coils 12 so that these adjacent coils are also powered in a transformer-like manner by mutual induction. In effect, one specific coil 12 is used to drive all of the various coils 12' solely by mutual induction between the coils. This method has several significant advantages. Firstly, only comparatively low voltages are required at the terminals of the individual coils since they are electrically in parallel; a series-wound coil of the same total number of turns would require many kilovolts of excitation voltage, with accompanying high-voltage insulation requirements, corona discharge problems, and safety hazards. Secondly, the circuits 26 are electrically isolated so that they do not all require exactly the same voltage, a requirement which would be necessary if all of the circuits 26 were connected to a common voltage source.

It is not to be assumed from the above discussion that the apparatus 10 requires the use of coils 12, 12' which are symmetrical relative to an axis. To increase the efficiency of the apparatus 10, it is desirable to construct all of the coils 12, 12' so that they are of an identical oval shape such that their interiors reasonably conform to the non-rectilinear shape of a human body. When the interiors of the coils are of such a configuration, a relatively large segment of the body can be located within them. This optional feature of the apparatus is important in the treatment of many internal neoplasms located within the trunk of the body. Even the introduction of the heart within an alternating magnetic field having a frequency as used in the present apparatus does not apparently affect the body to any significant or material extent. Thus the apparatus of the invention can be used to treat a neoplasm which is immediately adjacent to or is associated with the heart in one manner or another.

The amount of time that a part of the body is generally within the coils when using the present invention varies, of course, depending upon the total amount of heat or the maximum temperature required in order to cause necrosis of the neoplastic growth. When the magnetic particles employed in the hysteresis composition have a Curie point in the range $42^{\circ}C$ to $90^{\circ}C$, excessive overheating is normally prevented by the loss of magnetic properties of the particles used as they reach a temperature corresponding to their Curie points. When, however, magnetic particles are used which have Curie points above the specific temperature range noted, it is considered highly desirable to carefully monitor the time that a specific area containing magnetic particles is generally within the coils and the power supplied to these coils. Generally speaking, at a specific power level the coils should not be operated any longer than is necessary to cause necrosis of neoplastic tissue. Since this is dependent upon both temperature and time, it is desirable to carefully monitor the temperature in the area of treatment.

When using the apparatus, normal cells or tissue adjacent to the hysteresis composition will become heated to at least a degree and as a result such normal tissue or growth will frequently be necrosed. Normally, this does not present any significant problems. As is well known, the body has what may be regarded as inherent mechanisms for dealing with many different eventualities and is reasonably capable of disposing of both necrosed normal and neoplastic tissue or cells through normal metabolic processes. Further, the body is reasonably capable of developing what may be loosely referred to as scar tissue to compensate for normal growth which has been lost as a result of hyperthermia. Not infrequently, the body, through its own internal mechanisms, will develop barriers
of a known or conventional type isolating areas where
magnetic particles have been used from the normal
metabolic operation of the body.

Claims:

1. An induction heating apparatus which comprises an induction heating coil (12) and means (30,32) for passing an alternating current through the coil to cause induction heating therein, characterised in that at least one further coil (12') is provided adjacent and in axial alignment with the heating coil, the or each further coil (12') being electrically isolated from the means (30,32) for passing an alternating current and being connected to form a resonant circuit (26) arranged to resonate when alternating current passes through the heating coil (12) thereby to provide additional induction heating.

2. An induction heating apparatus according to claim 1, in which each coil (12) is connected to a capacitor means (28) to provide the resonant circuit in the form of a tank circuit (26).

3. An induction heating apparatus according to claim 1 or 2, in which the means (30,32) for passing an alternating current is adjustable to vary the frequency of the current.

4. An induction heating apparatus according to any of claims 1 to 3, in which the openings in the coils (12, 12') are of an identical oval shape reasonably corresponding to the configuration of a human body and are aligned with each other.

5. An induction heating apparatus according to any of claims 1 to 4, in which each coil (12, 12') comprises a hollow tube (14) in which is disposed a coil of multi-stranded wire (16), and further including means (18,20, 22,24) for circulating coolant fluid through the tubes (14).

6.      An induction heating apparatus according to any of claims 1 to 5, in which the coils (12,12') are identical in configuration.

7.      An induction heating apparatus comprising an induction heating coil (12) and means (30,32) for passing an alternating current through the coil to cause induction heating therein, characterised in that

(a)    at least one further coil (12') is provided adjacent to and in axial alignment with the heating coil (12),

(b)    the or each further coil (12') is electrically isolated from the means (30,32) for passing an alternating current through the heating coil (12) and is connected to a capacitor means (28) to provide a tank circuit (26) arranged to resonate when the alternating current passes through the heating coil (12), thereby providing additional induction heating,

(c)    each capacitor means (28) has the same capacitor value,

(d)    each tank circuit (26) has the same resonant frequency,

(e)    the or each further coil (12') is identical in configuration to the heating coil (12),

(f)    each coil (12,12') comprises a series of turns of a hollow electrically non-conducting tube (14) in which is loosely disposed a coil of multi-stranded conductor (16),

(g)    the apparatus includes means (18, 20, 22,24) for circulating coolant liquid through the interior of the tube (14), and

(h)    the means (30,32) for passing the alternating current through the heating coil (12) is capable of being adjusted so that the frequency of the alternating current can be maintained at the same value as the resonant frequency of the tank circuit (26).

FIG. 1

FIG. 2

22  24  10  18  16  1  12'  14  18  12'  14  16  18  14  12  28  16  18  14  12'  18  20  26  28  34  28  34  32  34  28  34  30  12  10  24  20  30

1/1

0208338